(19) Europäisches Patentamt
European Patent Office
Office européen des brevets

(11) Veröffentlichungsnummer: **0 120 393 A1**

(12) # EUROPÄISCHE PATENTANMELDUNG

(21) Anmeldenummer: 84102699.0

(22) Anmeldetag: 13.03.84

(51) Int. Cl.³: C 07 C 93/227
A 01 N 39/02, A 01 N 43/40

(30) Priorität: 23.03.83 JP 47027/83

(43) Veröffentlichungstag der Anmeldung: 03.10.84 Patentblatt 84/40

(84) Benannte Vertragsstaaten: AT BE CH DE FR GB IT LI NL

(71) Anmelder: NIHON TOKUSHU NOYAKU SEIZO K.K.
No.4, 2-chome, Nihonbashi Honcho Chuo-ku
Tokyo 103(JP)

(72) Erfinder: Aya, Masahiro
2-844, Suzuki-cho
Kodaira-shi Tokyo(JP)

(72) Erfinder: Saito, Junichi
3-7-12, Osawa
Mitaka-shi Tokyo(JP)

(72) Erfinder: Yasui, Kazuomi
7-6-15 Shakujii-dai
Nerima-ku Tokyo(JP)

(72) Erfinder: Shiokawa, Kozo
210-6, Shukugawara Tama-ku
Kawasaki-shi Kanagawa-ken(JP)

(72) Erfinder: Kamochi, Atsumi
2-24-10, Higashi-Toyoda
Hino-shi Tokyo(JP)

(74) Vertreter: Schumacher, Günter, Dr. et al,
c/o Bayer AG Konzernverwaltung RP Patentabteilung
D-5090 Leverkusen 1 Bayerwerk(DE)

(54) **Herbizide substituierte Phenoxyprepionsäureester.**

(57) Neue substituierte Phenoxypropionsäureester der Formel

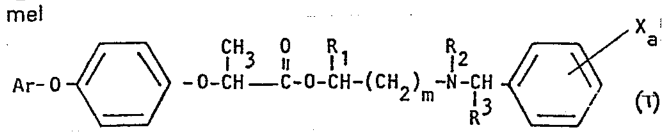

(I)

in welcher
Ar für die Reste der Formeln

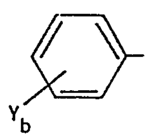 oder 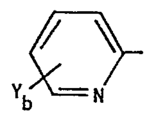 steht,

worin
Y für Halogen oder Trifluormethyl steht und
b für 1 oder 2 steht,
R¹ und R³ unabhängig voneinander für Wasserstoff oder Methyl stehen,
R² für niederes Alkyl oder niederes Alkenyl steht,
X für Wasserstoff, Halogen oder niederes Alkoxy steht,
m für 1 oder 2 steht und
a für 1 oder 2 steht,
mehrere Verfahren zur Herstellung dieser Stoffe und deren Verwendung als Herbizide, neue Zwischenprodukte und Verfahren zu ihrer Herstellung.

EP 0 120 393 A1

Croydon Printing Company Ltd.

0120393

NIHON TOKUSHU NOYAKU SEIZO K.K., Tokyo / Japan

Dü/Kü-c
Ib

BEZEICHNUNG GEÄNDERT
Siehe Titelseite

Substituierte Phenoxypropionsäureester

Die Erfindung betrifft neue substituierte Phenoxypropionsäureester, mehrere Verfahren zu deren Herstellung und deren Verwendung als Herbizide. Außerdem betrifft die Erfindung auch neue Zwischenprodukte und Verfahren zu ihrer Herstellung.

Es ist bereits bekannt, daß bestimmte 2-(4-Phenoxy-phenoxy)-propionsäure-Derivate herbizide Eigenschaften besitzen (vgl. DE-OS 2 617 804 und japanische Offenlegungsschrift Nr. 131, 545 - 1977). So kann zum Beispiel 2-/4-(4-Trifluormethyl-phenoxy)-phenoxy/-propionsäure-2-(dimethylamino)-ethyl-ester der Formel

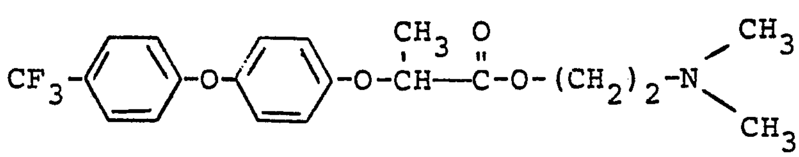

(A-1)

zur Bekämpfung von Unkräutern verwendet werden. Die Wirkung dieses Stoffes ist jedoch nicht immer völlig ausreichend.

Es wurden jetzt neue substituierte Phenoxypropionsäureester der Formel

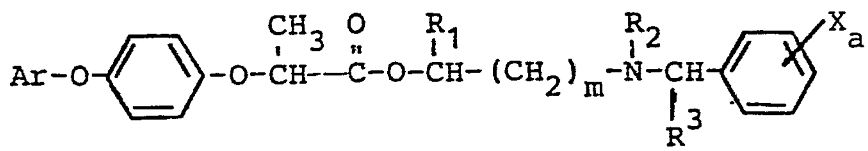

(I)

in welcher

Ar für die Reste der Formeln

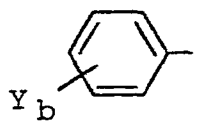 oder 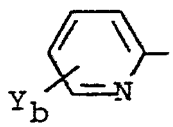 steht,

worin

Y für Halogen oder Trifluormethyl steht und

b für 1 oder 2 steht,

$R^1$ und $R^3$ unabhängig voneinander für Wasserstoff oder Methyl stehen,

$R^2$ für niederes Alkyl oder niederes Alkenyl steht,

X für Wasserstoff, Halogen oder niederes Alkoxy steht,

m für 1 oder 2 steht und

a für 1 oder 2 steht,

gefunden.

Weiterhin wurde gefunden, daß man die neuen substituierten Phenoxypropionsäureester der Formel (I) erhält, wenn man

a) Verbindungen der Formel

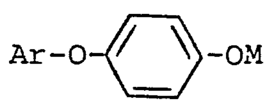

(II)

in welcher

Ar die oben angegebene Bedeutung hat

und

M für Wasserstoff oder ein Alkalimetallatom steht,

mit Verbindungen der Formel

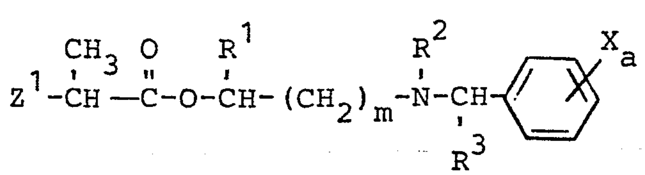

(III)

in welcher

$R^1$, $R^2$, $R^3$, X, a und m die oben angegebenen Bedeutungen haben und

$Z^1$ für Halogen steht,

umsetzt,

oder

b) Verbindungen der Formel

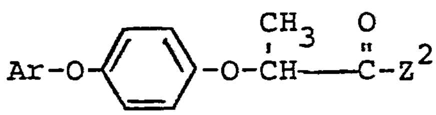

(IV)

in welcher

Ar die oben angegebene Bedeutung hat

und

$Z^2$ für Hydroxy oder Halogen steht

mit Verbindungen der Formel

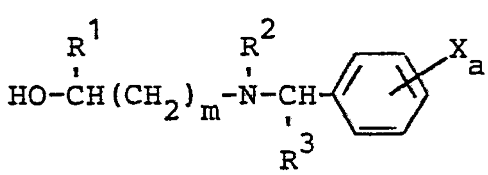

(V)

in welcher

Nit 159

$R^1$, $R^2$, $R^3$, X, a und m die oben angegebenen Bedeutungen haben,

umsetzt,

oder

c) Verbindungen der Formel

$$Ar\text{-}Z^1 \qquad (VI)$$

in welcher

Ar und $Z^1$ die oben angegebenen Bedeutungen haben,

mit Verbindungen der Formel

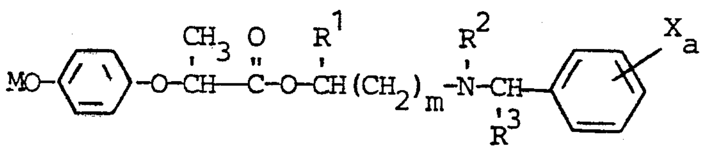

(VII)

in welcher

$R^1$, $R^2$, $R^3$, X, M, a und m die oben angegebenen Bedeutungen haben,

umsetzt.

Schließlich wurde gefunden, daß sich die erfindungsgemäßen substituierten Phenoxypropionsäureester der Formel (I) durch sehr gute herbizide Eigenschaften auszeichnen. Die vorliegende Erfindung betrifft deshalb auch herbizide Mittel, die substituierte Phenoxypropionsäureester der Formel (I) als Wirkstoffe enthalten. Außerdem betrifft die vorliegende Erfindung ein Verfahren zur Bekämpfung von Unkräutern, welches darin besteht, daß man substituierte Phenoxypropionsäureester der Formel (I) auf die Pflanzen und/oder deren Lebensraum ausbringt.

Überraschenderweise besitzen die erfindungsgemäßen Stoffe der Formel (I), eine ausgezeichnete herbizide Wirksamkeit. Sie sind besonders vorteilhaft einsetzbar zur selektiven Bekämpfung einer großen Anzahl von Unkräutern, wie zum Beispiel Unkräuter aus der Familie der Graminaceae, ohne eine nennenswerte Phytoxizität gegenüber landwirtschaftlichen Nutzpflanzen zu entfalten. Es ist besonders überraschend, daß die erfindungsgemäßen Verbindungen der Formel (I) auch eine bessere herbizide Wirksamkeit aufweisen als der 2-/4-(4-Trifluormethylphenoxy)-phenoxy/-propionsäure-2-(dimethylamino)-ethylester, welches ein konstitutionell ähnlicher, aus dem Stand der Technik bekannter Wirkstoff gleicher Wirkungsart ist. Vor allem ist hervorzuheben, daß die erfindungsgemäßen substituierten Phenoxypropionsäureester der Formel (I) auch dann, wenn sie in niedrigen Aufwandmengen eingesetzt werden, eine hervorragende Selektivität besitzen und außerdem auch der Regenerierung von Unkräutern, insbesondere von perennierenden grasartigen Unkräutern,

infolge ihrer ausgezeichneten Langzeitwirkung über eine lange Zeitdauer hinweg erfolgreich entgegenwirken.

Die erfindungsgemäßen Verbindungen der Formel (I) sind strukturell dadurch charakterisiert, daß die Alkylgruppe in einem substituierten Phenoxypropionsäure-alkylester zusätzlich noch durch eine substituierte Benzylamino-Gruppe substituiert ist.

Bevorzugte erfindungsgemäße Wirkstoffe sind diejenigen substituierten Phenoxypropionsäureester der Formel (I), in denen

Ar für die Reste der Formeln

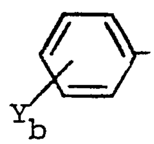 oder 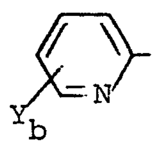 steht, worin

Y für Fluor, Chlor, Brom, und/oder für Trifluormethyl steht und

b für 1 oder 2 steht,

$R^1$ und $R^3$ unabhängig voneiander für Wasserstoff oder Methyl stehen,

$R^2$ für Alkyl mit 1 bis 4 Kohlenstoffatomen, wie zum Beispiel Methyl, Ethyl, Propyl, Isopropyl, n-Butyl, iso-Butyl, sek.-Butyl oder tert.-Butyl, oder für Alkenyl mit 2 oder 3

Kohlenstoffatomen, wie zum Beispiel für Vinyl, Allyl oder 1-Propenyl steht,

X für Wasserstoff, Fluor, Chlor, Brom oder Alkoxy mit 1 bis 4 Kohlenstoffatomen, wie zum Beispeil Methoxy, Ethoxy, Propoxy, Isopropoxy, n-Butoxy, iso-Butoxy, sek.-Butoxy oder tert.-Butoxy, steht,

m für 1 oder 2 steht und

a für 1 oder 2 steht.

Der Verlauf des erfindungsgemäßen Verfahrens (a) kann durch das folgende Reaktionsschema veranschaulicht werden:

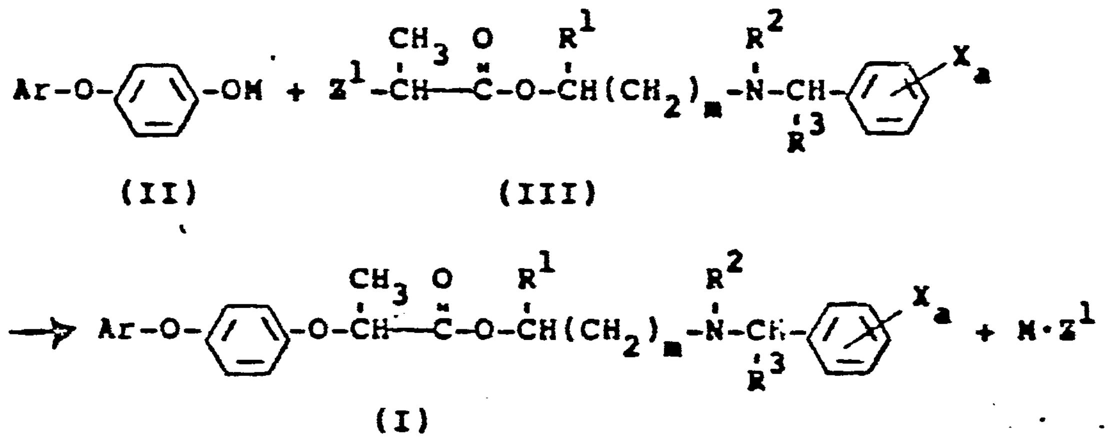

(II) (III)

(I)

Verwendet man speziell 4-(4-Trifluormethylphenoxy)-phenol und 2-Brom-propionsäure-2-(N-methyl-benzyl-amino)-ethylester als Ausgangsstoffe, so kann der Verlauf des erfindungsgemäßen Verfahrens (a) durch das folgende Reaktionsschema wiedergegeben werden:

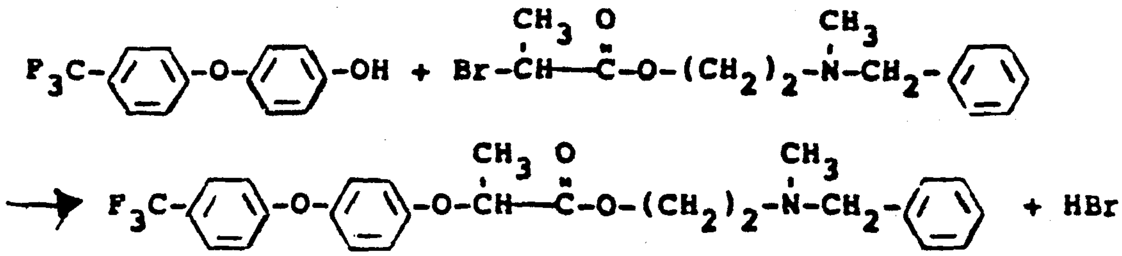

Die bei dem erfindungsgemäßen Verfahren (a) als Ausgangsstoffe benötigten Verbindungen sind durch die Formel (II) eindeutig definiert. In dieser Formel steht Ar vorzugsweise für diejenigen Reste, die bereits im Zusammenhang mit der Beschreibung der erfindungsgemäßen Stoffe der Formel (I) vorzugsweise für diesen Rest genannt wurden. M steht vorzugsweise für Wasserstoff, Lithium, Natrium und Kalium.

Als Beispiele für Verbindungen der Formel (II) seien genannt:
4-(4-Trifluormethyl-phenoxy)-phenol,
4-(5-Trifluormethyl-pyridyl-2-oxy)-phenol,
4-(3,5-Dichlor-pyridyl-2-oxy)-phenol und
4-(3-Chlor-5-trifluormethyl-pyridyl-2-oxy)-phenol
sowie die entsprechenden Lithium-, Natrium- und Kalium-Salze.

Die bei dem erfindungsgemäßen Verfahren (a) weiterhin als Ausgangsstoffe benötigten Verbindungen sind durch die Formel (III) allgemein definiert. In dieser Formel stehen $R^1$, $R^2$, $R^3$, X, a und m vorzugsweise für diejenigen Reste und Indices, die bereits im Zusammenhang mit der

Beschreibung der erfindungsgemäßen Stoffe der Formel (I) vorzugsweise für diese Reste und Indices genannt wurden. $Z^1$ steht vorzugsweise für Chlor oder Brom.

Als Beispiele für Verbindungen der Formel (III) seien genannt:

2-Chlor(oder Brom)-propionsäure-2-(N-methyl-benzyl-amino)-ethylester,

2-Chlor(oder Brom)-propionsäure-2-(N-isopropyl-benzyl-amino)-ethylester,

2-Chlor(oder Brom)-propionsäure-2-(N-methyl-α-methyl-benzyl-amino)-ethylester,

2-Chlor(oder Brom)-propionsäure-2-(N-methyl-2-fluorbenzyl-amino)-ethylester,

2-Chlor(oder Brom)-propionsäure-2-(N-allyl-4-chlor-benzyl-amino)-ethylester,

2-Chlor(oder Brom)-propionsäure-2-(N-methyl-4-methoxy-benzyl-amino)-ethylester,

2-Chlor(oder Brom)-propionsäure-2-(N-isopropyl-3,4-dichlorbenzyl-amino)-ethylester,

2-Chlor(oder Brom)-propionsäure-2-(N-methyl-4-chlor-benzyl-amino)-ethylester,

2-Chlor(oder Brom)-propionsäure-1-(methyl)-2-(N-methylbenzyl-amino)-ethylester und

2-Chlor(oder Brom)-propionsäure-3-(N-methylbenzyl-amino)-propylester.

Die Verbindungen der Formel (III) sind bisher noch nicht in der Literatur beschrieben worden. Daher betrifft die vorliegende Erfindung auch neue Verbindungen der Formel (III).

0120893

Außerdem betrifft die Erfindung auch ein Verfahren zur Herstellung der Verbindungen der Formel (III). Man erhält diese Verbindungen der Formel (III), indem man

d) Verbindungen der Formel

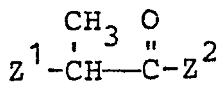

(VIII)

in welcher

$Z^1$ und $Z^2$ die oben angegebenen Bedeutungen haben,

mit Verbindungen der Formel

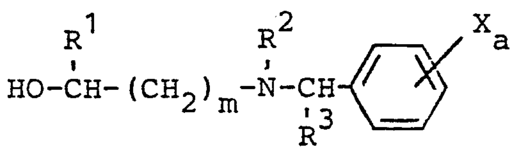

(V)

in welcher

$R^1$, $R^2$, $R^3$, X, a und m die oben angegebenen Bedeutungen haben,

umsetzt.

Die Umsetzung nach dem erfindungsgemäßen Verfahren (a) wird vorzugsweise in Gegenwart eines Lösungsmittels oder eines Verdünnungsmittels durchgeführt. Zu diesem Zweck können alle inerten Solventien und Verdünnungsmittel verwendet werden.

Beispiele für solche Lösungsmittel oder Verdünnungsmittel sind Wasser; aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Tetrachlorkohlenstoff, Ethylenchlorid, Trichlorethylen und Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropyolketon und Methylisobutylketon; Nitrile wie Acetonitril, Propionitril und Acrylnitril; Alkohole wie Methanol, Ethanol, Isopropanol, Butanol und Ethylenglykol; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Die Umsetzung nach dem erfindungsgemäßen Verfahren (a) kann innerhalb eines größeren Temperturbereiches durchgeführt werden. Im allgemeinen arbeitet man bei Temperaturen zwischen -20°C und dem Siedepunkt des Reaktionsgemisches, vorzugsweise zwischen 0°C und 100°C.

Die Umsetzung nach dem erfindungsgemäßen Verfahren (a) wird vorzugsweise unter atmosphärischem Druck durchgeführt; sie kann jedoch auch unter erhöhtem oder vermindertem Druck vorgenommen werden.

Der Verlauf des erfindungsgemäßen Verfahrens (b) kann durch das folgende Reaktionsschema veranschaulicht werden:

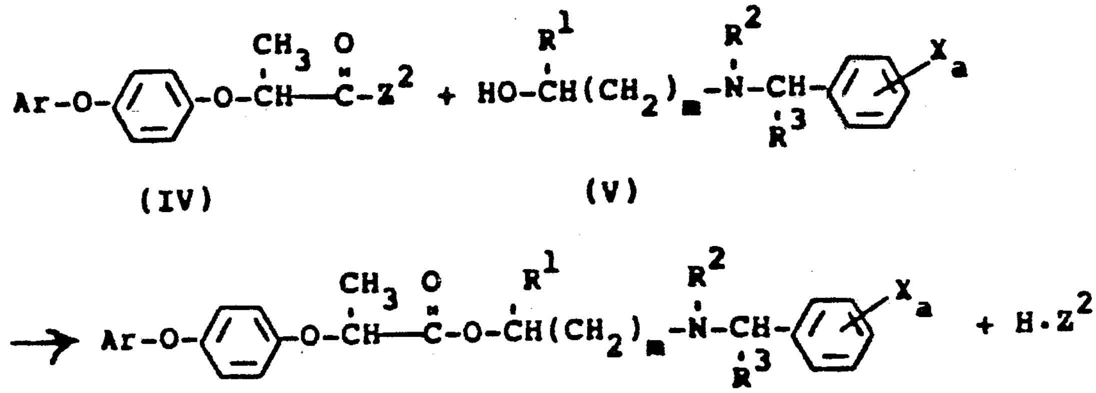

(IV) (V)

(I)

Verwendet man speziell 2-[4-(3,5-Dichlorpyridyl-2-oxy)-phenoxy]-propionylchlorid und 2-(N-Isopropyl-benzyl-amino)-ethanol als Ausgangsstoffe, so kann der Verlauf des erfindungsgemäßen Verfahrens (b) durch das folgende Reaktionsschema (b) wiedergegeben werden:

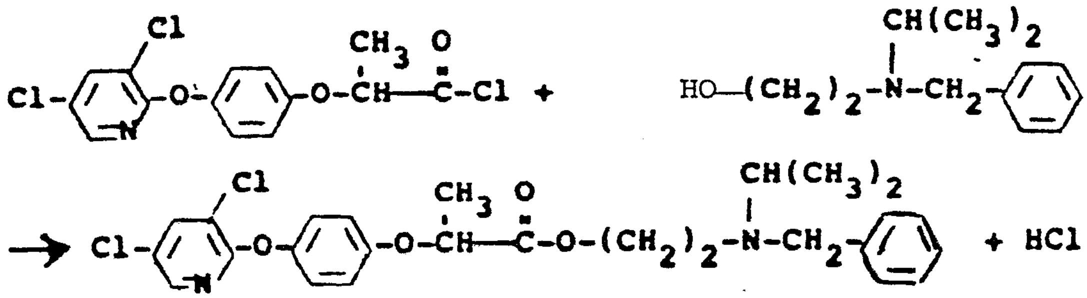

Die bei dem erfindungsgemäßen Verfahren (b) als Ausgangsstoffe benötigten Verbindungen sind durch die Formel (IV) allgemein definiert. In dieser Formel hat Ar vorzugsweise diejenigen Bedeutungen, die bereits im Zusammenhang mit der Beschreibung der erfindungsgemäßen Stoffe der Formel (I) für diesen Rest vorzugsweise genannt wurden. $Z^2$ steht vorzugsweise für Hydroxyl, Chlor und Brom.

Als Beispiele für Verbindungen der Formel (IV) seien genannt:
2-/4-(4-Trifluormethyl-phenoxy)-phenoxy/-propionylchlorid,
2-/4-(5-Trifluormethyl-pyridyl-2-oxy)-phenoxy/-propionylchlorid,
2-/4-(3,5-Dichlor-pyridyl-2-oxy)-phenoxy/-propionylchlorid und
2-/4-(3-Chlor-5-trifluormethyl-pyridyl-2-oxy)-phenoxy/-propionylchlorid
sowie die entsprechenden Bromide und freien Propionsäuren.

Die bei dem erfindungsgemäßen Verfahren (b) weiterhin als Ausgangsstoffe benötigten Verbindungen sind durch die Formel (V) allgemein definiert. In dieser Formel haben $R^1$, $R^2$, $R^3$, X, a und m vorzugsweise diejenigen Bedeutungen, die bereits im Zusammenhang mit der Beschreibung der erfindungsgemäßen Stoffe der Formel (I) vorzugsweise für diese Reste und Indices genannt wurden.

Als Beispiele für Verbindungen der Formel (V) seien genannt:
2-(N-Methyl-benzylamino)-ethanol,
2-(N-Isopropyl-benzylamino)-ethanol,
2-(N-Methyl-α-methylbenzyl-amino)-ethanol,

2-(N-Methyl-2-fluorbenzyl-amino)-ethanol,
2-(N-Allyl-4-chlorbenzyl-amino)-ethanol,
2-(N-Methyl-4-methoxybenzyl-amino)-ethanol,
2-(N-Isopropyl-3,4-dichlorbenzyl-amino)-ethanol,
3-(N-Methyl-4-chlorbenzyl-amino)-ethanol,
1-(N-Methyl-benzylamino)-2-propanol
und
3-(N-Methyl-benzylamino)-1-propanol.

Bei der Durchführung des erfindungsgemäßen Verfahrens (b) können mit Ausnahme von Alkoholen vorzugsweise alle diejenigen inerten Solventien und Verdünnungsmittel verwendet werden, die bereits im Zusammenhang mit der Durchführung des erfindungsgemäßen Verfahrens (a) genannt wurden. Die Endprodukte werden dabei in hoher Ausbeute und sehr guter Reinheit erhalten. Ebenso können bei der Durchführung des erfindungsgemäßen Verfahrens (b) vorzugsweise alle diejenigen Säurebindemittel eingesetzt werden, die bereits im Zusammenhang mit der Beschreibung des erfindungsgemäßen Verfahrens (a) genannt wurden.

Die Umsetzung nach dem erfindungsgemäßen Verfahren (b) kann innerhalb eines größeren Temperaturbereiches durchgeführt werden. Im allgemeinen arbeitet man bei Temperaturen zwischen -20°C und dem Siedepunkt des Reaktionsgemisches, vorzugsweise zwischen 0°C und 100°C.

Die Umsetzung nach dem erfindungsgemäßen Verfahren (b) wird vorzugsweise unter atmosphärischem Druck durchdurchgeführt, sie kann jedoch auch unter erhöhtem oder vermindertem Druck vorgenommen werden.

Der Verlauf des erfindungsgemäßen Verfahrens (c) kann durch das folgende Reaktionsschema veranschaulicht werden:

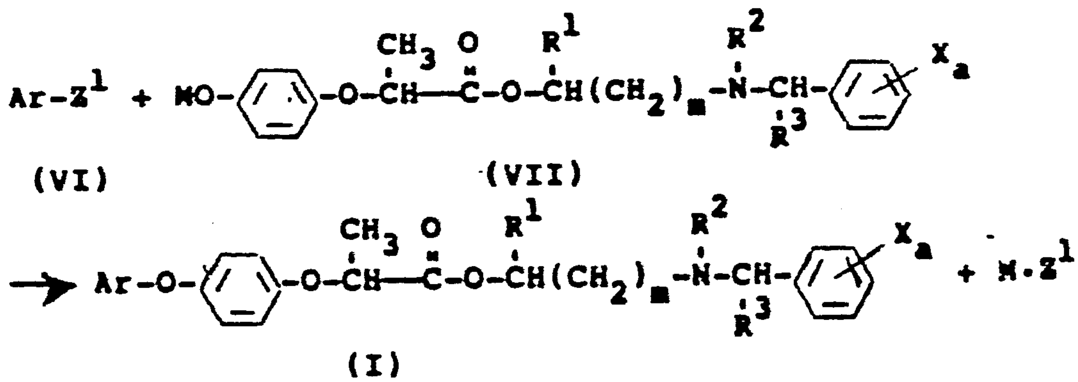

(VI) (VII)

(I)

Verwendet man speziell 2-Brom-5-trifluormethyl-pyridin und 2-(4-Hydroxy-phenoxy)-propionsäure-2-(N-methyl-benzylamino)-ethylester als Ausgangsstoffe, so kann der Verlauf des erfindungsgemäßen Verfahrens (c) durch das folgende Reaktionsschema wiedergegeben werden:

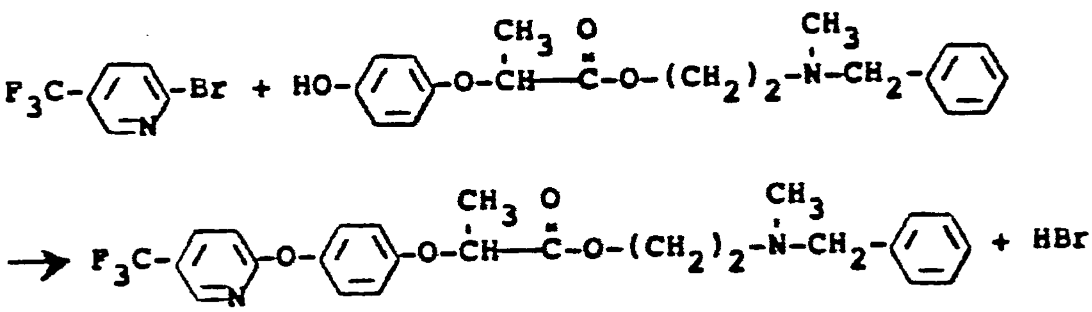

Die bei dem erfindungsgemäßen Verfahren (c) als Ausgangsstoffe benötigten Verbindungen sind durch die Formel (VI) allgemein definiert. In dieser Formel hat Ar vorzugsweise diejenigen Bedeutungen, die bereits im Zusammenhang mit der Beschreibung der erfindungsgemäßen Stoffe der Formel (I) für diesen Rest vorzugsweise genannt wurden. $Z^1$ steht vorzugsweise für Fluor, Chlor und Brom.

Als Beispiele für Verbindungen der Formel (VI) seien genannt:
4-Chlor(oder Brom)-trifluormethylbenzol,
2-Chlor(oder Fluor, oder Brom)-5-trifluormethylpyridin,
2,3,5-Trichlorpyridin,
2-Brom-3,5-dichlorpyridin,
2,3-Dichlor-5-trifluormethyl-pyridin und 2-Brom-3-Chlor-5-trifluormethyl-pyridin.

Die bei dem erfindungsgemäßen Verfahren (c) weiterhin als Ausgangsstoffe benötigten Verbindungen sind durch die Formel (VII) allgemein definiert. In dieser Formel haben $R^1$, $R^2$, $R^3$, X, a und m vorzugsweise diejenigen Bedeutungen, die bereits im Zusammenhang mit der Beschreibung der erfindungsgemäßen Stoffe der Formel (I) vorzugsweise für diese Reste und Indices genannt wurden. M steht vorzugsweise für Wasserstoff, Lithium, Natrium und Kalium.

Als Beispiele für Verbindungen der Formel (VII) seien genannt:

2-(4-Hydroxyphenoxy)-propionsäure-2-(N-methyl-benzyl-amino)-ethylester,
2-(4-Hydroxy-phenoxy)-propionsäure-2-(N-isopropyl-benzyl-amino)-ethylester,
2-(4-Hydroxy-phenoxy)-propionsäure-2-(N-methyl-α-methylbenzyl-amino)-ethylester,
2-(4-Hydroxy-phenoxy)-propionsäure-2-(N-methyl-2-fluorbenzyl-amino)-ethylester,
2-(4-Hydroxy-phenoxy)-propionsäure-2-(N-allyl-4-chlorbenzyl-amino)-ethylester,
2-(4-Hydroxy-phenoxy)-propionsäure-2-(N-methyl-4-methoxy-benzyl-amino)-ethylester,
2-(4-Hydroxy-phenoxy)-propionsäure-2-(N-isopropyl-3,4-dichlor-benzyl-amino)-ethylester,
2-(4-Hydroxy-phenoxy)-propionsäure-2-(N-methyl-4-chlor-benzyl-amino)-ethylester,
2-(4-Hydroxy-phenoxy)-propionsäure-1-methyl-2-(N-methyl-benzyl-amino)-ethylester und
2-(4-Hydroxy-phenoxy)-propionsäure-3-(N-methyl-benzyl-amino)-propylester

sowie die entsprechenden Lithium-, Natrium- und Kalium-Salze.

Die Verbindungen der Formel (VII) sind bisher noch nicht in der Literatur beschrieben worden. Daher betrifft die vorliegende Erfindung auch neue Verbindungen der Formel (VII).

Außerdem betrifft die Erfindung auch ein Verfahren zur Herstellung der Verbindungen der Formel (VII). Man erhält diese Verbindungen der Formel (VII), indem man

e) Verbindungen der Formel

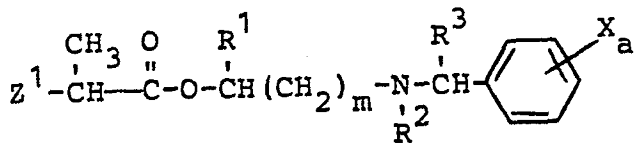

(III)

in welcher

$R^1$, $R^2$, $R^3$, X, $Z^1$, a und m die oben angegebenen Bedeutungen haben,

mit Hydrochinon der Formel

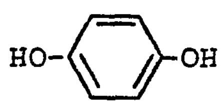

(IX)

umsetzt und gegebenenfalls die dabei entstehenden Produkte mit einem Alkalimetallhydroxid, einem Alkalimetallcarbonat oder einem Alkalimetallalkoholat umsetzt.

Bei der Durchführung des erfindungsgemäßen Verfahrens (c) können mit Ausnahme von Alkoholen vorzugsweise alle diejenigen inerten Solventien und Verdünnungsmittel verwendet werden, die bereits im Zusammenhang

0120393

mit der Durchführung des erfindungsgemäßen Verfahrens (a) genannt wurden. Die Endprodukte werden dabei in hoher Ausbeute und sehr guter Reinheit erhalten. Ebenso können bei der Durchführung des erfindungsgemäßen Verfahrens (c) vorzugsweise alle diejenigen Säurebindemitel eingesetzt werden, die bereits im Zusammenhang mit der Beschreibung des erfindungsgemäßen Verfahrens (a) genannt wurden.

Die Umsetzung nach dem erfindungsgemäßen Verfahren (c) kann innerhalb eines größeren Temperaturbereiches durchgeführt werden. Im allgemeinen arbeitet man bei Temperaturen zwischen -20°C und dem Siedepunkt des Reaktionsgemisches, vorzugsweise zwischen 0°C und 100°C.

Die Umsetzung nach dem erfindungsgemäßen Verfahren (c) wird vorzugsweise unter atmosphärischem Druck durchgeführt; sie kann jedoch auch unter erhöhtem oder vermindertem Druck vorgenommen werden.

Der Verlauf des erfindungsgemäßen Verfahrens (d) kann durch das folgende Reaktionsschema veranschaulicht werden:

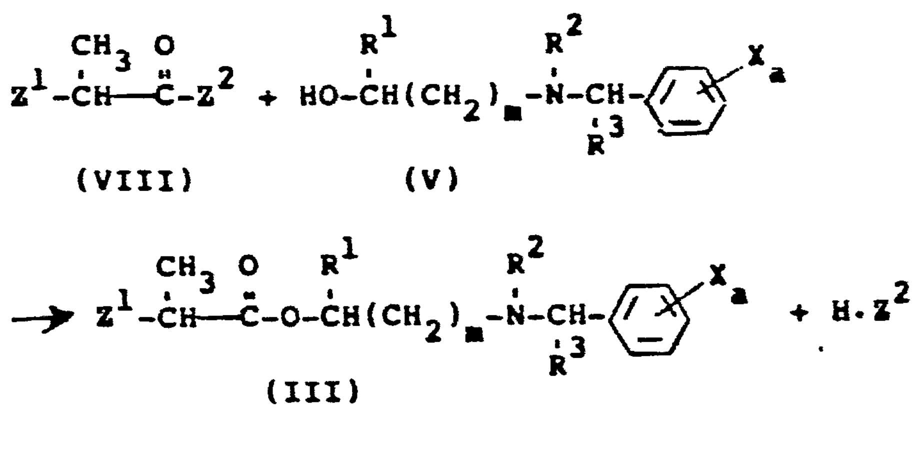

(VIII) (V)

(III)

Verwendet man speziell 2-Brom-propionyl-bromid und 2-(N-Methyl-benzyl-amino)-ethanol als Ausgangsstoffe, so kann der Verlauf des erfindungsgemäßen Verfahrens (d) durch das folgende Reaktionsschema wiedergegeben werden:

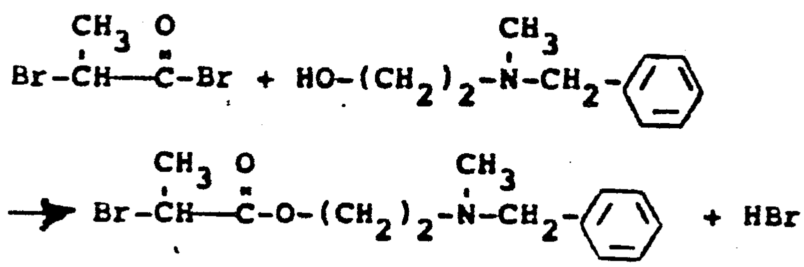

Die bei dem erfindungsgemäßen Verfahren (d) als Ausgangsstoffe benötigten Verbindungen sind durch die Formel (VIII) allgemein definiert. In dieser Formel steht $Z^1$ vorzugsweise für Chlor und Brom. $Z^2$ steht vorzugsweise für Hydroxyl, Chlor oder Brom.

Spezielle Beispiele für Verbindungen der Formel (VIII) sind 2-Chlorpropionsäure, 2-Brompropionsäure sowie die entsprechenden Chloride und Bromide.

Bei der Durchführung des erfindungsgemäßen Verfahrens (d) können mit Ausnahme von Alkoholen vorzugsweise alle diejenigen inerten Solventien und Verdünnungsmittel verwendet werden, die bereits im Zusammenhang mit dem erfindungsgemäßen Verfahren (a) genannt wurden. Die Zwischenprodukte der Formel (III) werden dabei in hoher Ausbeute und sehr guter Reinheit erhalten.

Ebenso können bei der Durchführung des erfindungsgemäßen Verfahrens (d) vorzugsweise alle diejenigen Säurebindemittel eingesetzt werden, die bereits im Zusammenhang mit der Beschreibung des erfindungsgemäßen Verfahrens (a) genannt wurden.

Die Umsetzung nach dem erfindungsgemäßen Verfahren (d) kann innerhalb eines größeren Temperaturbereiches durchgeführt werden. Im allgemeinen arbeitet man bei Temperaturen zwischen -20°C und dem Siedepunkt des Reaktionsgemisches, vorzugsweise zwischen 0°C und 100°C.

Die Umsetzung nach dem erfindungsgemäßen Verfahren (d) wird vorzugsweise unter atmosphärischem Druck durchgeführt; sie kann jedoch auch unter erhöhtem oder vermindertem Druck vorgenommen werden.

Der Verlauf des erfindungsgemäßen Verfahrens (e) kann durch das folgende Reaktionsschema veranschaulicht werden:

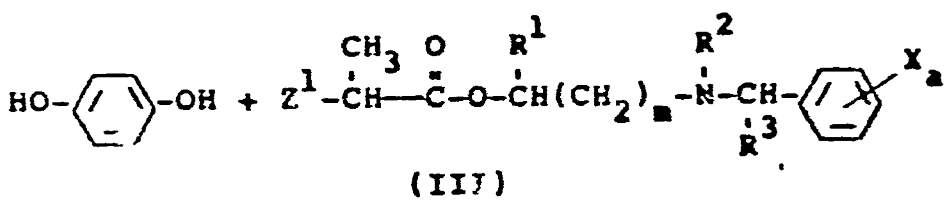

(III)

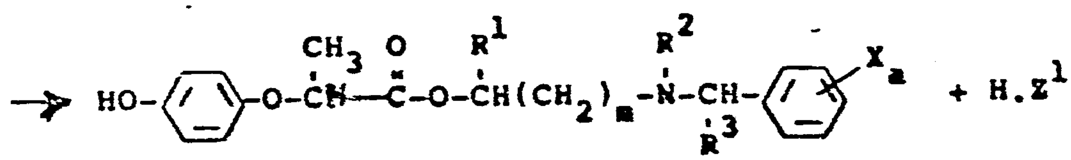

(VII-i)

Verwendet man speziell 2-Brompropionsäure-2-(N-methyl-benzyl-amino)-ethylester und Hydrochinon als Ausgangsstoffe, so kann der Verlauf des erfindungsgemäßen Verfahrens (e) durch das folgende Reaktionsschema wiedergegeben werden:

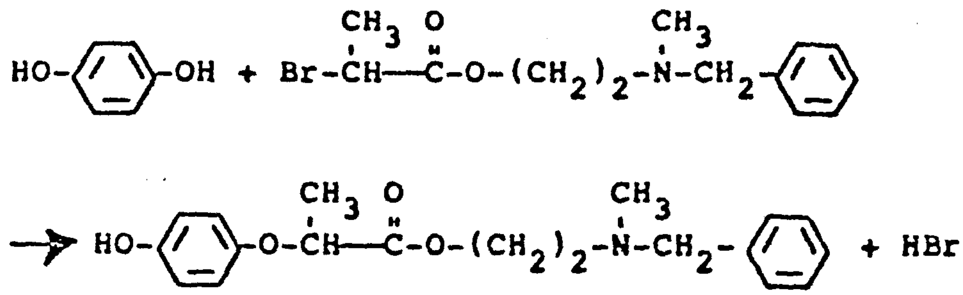

Die Verbindungen der Formel (VII), in denen M für ein Alkalimetallatom steht, können hergestellt werden, indem man 2-(4-Hydroxy-phenoxy)-propionsäureester der Formel (VII-i) mit einem Alkalimetallhydroxid, einem Alkalimetallcarbonat oder einem Alkalimetallalkoholat umsetzt.

Spezielle Beispiele für Alkalimetallhydroxide sind Lithiumhydroxid, Natriumhydroxid und Kaliumhydroxid. Spezielle Beispiele für Alkalimetallcarbonate sind Natriumcarbonat und Kaliumcarbonat. Spezielle Beispiele für Alkalimetallalkoholate sind Natrium- oder Kaliummethylat und Natrium- oder Kaliumethylat.

Bei der Durchführung des erfindungsgemäßen Verfahrens (e) können alle diejenigen inerten Solventien und

Verdünnungsmittel verwendet werden, die bereits im Zusammenhang mit der Beschreibung des erfindungsgemäßen Verfahrens (a) genannt wurden. Die Zwischenprodukte der Formel (VII) werden dabei in hoher Ausbeute und sehr guter Reinheit erhalten.

Die Umsetzung nach dem erfindungsgemäßen Verfahren (e) kann innerhalb eines größeren Temperaturbereiches durchgeführt werden. Im allgemeinen arbeitet man bei Temperaturen zwischen -20°C und dem Siedepunkt des Reaktionsgemisches, vorzugsweise zwischen 0°C und 100°C.

Die Umsetzung nach dem erfindungsgemäßen Verfahren (e) wird vorzugsweise unter atmosphärischem Druck durchgeführt; sie kann jedoch auch unter erhöhtem oder vermindertem Druck vorgenommen werden.

Die erfindungsgemäßen Verbindungen der Formel (I) besitzen ausgezeichnete selektive herbizide Eigenschaften. Sie können entweder vor oder nach dem Auflaufen der Pflanzen angewandt werden. Vorzugsweise werden sie vor dem Auflaufen der Pflanzen eingesetzt, das heißt nach dem pre-emergence-Verfahren. Sie können auch vor der Saat in den Boden eingearbeitet werden.

Da die erfindungsgemäßen Wirkstoffe nur eine niedrige oder gar keine Toxizität gegenüber warmblütigen Tieren

besitzen und eine gute Selektivität für Kulturpflanzen besitzen, also bei Anwendung in den üblichen Konzentrationen keine Phytotoxizität gegenüber Kulturpflanzen aufweisen, können sie vorteilhaft als Herbizide zur Unkrautbekämpfung eingesetzt werden, insbesondere zur Bekämpfung von grasartigen Unkräutern. Unter Unkräutern im weitesten Sinne sind hier alle Pflanzen zu verstehen, die an Plätzen wachsen, wo sie unerwünscht sind.

Die erfindungsgemäßen Wirkstoffe können zum Beispiel zur Bekämpfung folgender Pflanzen eingesetzt werden:
Echinochloa crus-galli P. Beauv.,
Digitaria adscendens Henr.,
Eleusine indica Gaertn.,
Setaria viridis P. Beauv.,
Avena fatua,
Alopecurus aequalis var. amurensis Ohwi,
Setaria lutescens,
Agropyron repens und
Agropyron tsukushiense var. transiens Ohwi.

Außerdem zeigen die erfindungsgemäßen Stoffe der Formel (I) auch eine ausgezeichnete herbizide und eine den Wiederauflauf hemmende Wirkung gegenüber perennierenden Unkräutern wie Johnson-Gras und Cynodon dactylon Persoon.

Die erfindungsgemäßen Wirkstoffe können in vielen Kulturen als selektive Herbizide eingesetzt werden.

Als Beispiele seien die folgenden Kulturen genannt:

Bohnen, Baumwolle, Möhren, Kartoffeln, Rüben, Kohlgemüse, Senf, Erdnüsse, Rettich, Tabak, Tomaten und Gurken.

Für den Einsatz als Herbizide können die Verbindungen gemäß der vorliegenden Erfindung zu verschiedenen Wirkstoffpräparaten formuliert werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Gebrauch werden die herbiziden Mittel in Form ihrer verschiedenen Präparate entweder unmittelbar oder nach dem Verdünnen mit Wasser auf die gewünschte Konzentration eingesetzt.

Beispiele für die landwirtschaftlich unbedenklichen Hilfsstoffe, auf die hier Bezug genommen wird, sind Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe /z.B. n-Hexan, Petrolether, Naphtha, Erdöl-Fraktionen (z.B. Paraffinwachse, Kerosin, Leichtöle, Mittelöle, Schweröle), Benzol, Toluol und Xylole7, halogenierte

Kohlenwasserstoffe (z.B. Methylenchlorid, Tetrachlorkohlenstoff, Trichlorethylen, Ethylenchlorid, Ethylendichlorid, Chlorbenzol und Chloroform), Alkohole (z.B. Methylalkohol, Ethylalkohol, Propylalkohol und Ethylenglykol), Ether (z.B. Ethylether, Ethylenoxid und Dioxan), Alkohol-ether (z.B. Ethylenglykol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für die oberflächenaktiven Mittel umfassen anionische oberflächenaktive Mittel wie Alkylschwefelsäureester (z.B. Natriumlaurylsulfat), Arylsulfonsäure-Salze (z.B. Alkylarylsulfonsäure-Salze und Natriumalkylnaphthalinsulfonate), Bernsteinsäure-Salze und Salze von Schwefelsäureestern von Polyethylenglykol-alkylarylethern, kationische oberflächenaktive Mittel wie Alkylamine (z.B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglykolether (z.B. Polyoxyethylenalkylarylether und deren Kondensationsprodukte), Polyoxyethylenglykolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), wirkungsverlängernde Mittel, Dispersions-Stabilisatoren (z.B. Casein, Tragant, Carboxymethylcellulose und Polyvinylalkohol) und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Beispiele für solche Anwendungsformen sind emulgierbare Konzentrate, Öle, benetzbare Pulver, lösliche Pulver, Suspensionen, Stäubemittel, Granulate und pulvrige Mittel.

Die Wirkstoffmenge kann in den anwendungsfertigen Formulierungen innerhalb eines größeren Bereiches variiert werden. Im allgemeinen liegt die Wirkstoffkonzentration zwischen 0,001 und 100 Gewichtsprozent, vorzugsweise zwischen 0,005 und 95 Gewichtsprozent.

Für den praktischen Gebrauch beträgt die geeignete Menge der aktiven Verbindung in den vorgenannten Mitteln in ihren verschiedenen Formen und gebrauchsfertigen Präparaten beispielsweise etwa 0,01 bis etwa 95 Gew.-%, vorzugsweise etwa 0,05 bis etwa 60 Gew.-%.

Der Gehalt des Wirkstoffs kann in geeigneter Weise je nach der Form des Präparats oder Mittels, dem Verfahren, Zweck, der Zeit und dem Ort seiner Anwendung, dem Zustand des Auftretens der Unkräuter etc. variiert werden.

Erforderlichenfalls können die Verbindungen gemäß der vorliegenden Erfindung weiterhin auch in Kombination mit anderen Agrochemikalien verwendet werden, beispielsweise mit Insektiziden, Fungiziden, Mitiziden, Nematiziden, Anti-Virus-Mitteln, anderen Herbiziden, Pflanzen-Wachstumsregulatoren und Lockstoffe /Organophosphorester-Verbindungen, Carbamat-Verbindungen, Dithio(oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen/ und/oder Düngemitteln.

Die erfindungsgemäße Wirkstoffe enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Dispergieren (Versprühen von Flüssigkeiten, Vernebeln, Zerstäuben, Stäuben, Streuen von Granulat, Wasser-Oberflächenbehandlung und Gießen) und Bodenbehandlung (Vermischen mit dem Boden und Streuen). Sie können auch mittels des sogenannten Ultra-Low-Volume-Sprühverfahrens aufgebracht werden. Nach diesem Verfahren kann der Wirkstoff sogar in einer Konzentration von 100 % zur Anwendung gelangen.

Die Aufwandmenge an Wirkstoff pro Flächeneinheit beträgt beispielsweise etwa 0,01 bis etwa 2,0 kg/ha, vorzugsweise etwa 0,05 bis etwa 1,0 kg/ha. In besonders gelagerten Fällen können oder sollten sogar die Aufwandmengen außerhalb des angegebenen Bereichs liegen.

Gemäß der vorliegenden Erfindung kann ein herbzides Mittel zur Verfügung gestellt werden, das als Wirkstoff die Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel etc. enthält.

Die vorliegende Erfindung betrifft weiterhin ein Verfahren zur Unkrautbekämpfung, das darin besteht, daß eine Verbindung der allgemeinen Formel (I) allein oder in Form einer Mischung mit einem Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel, einem synergistischen Mittel etc. auf Unkräuter und/oder ihren Lebensraum aufgebracht wird.

Die vorliegende Erfindung wird durch die folgenden Beispiele näher erläutert, ist jedoch nicht auf diese beschränkt.

Herstellungsbeispiele

Beispiel 1 (Zwischenprodukt)

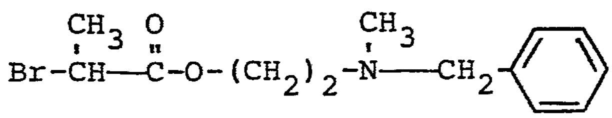

(Verbindung Nr. III-1)

Eine Lösung von 21,6 g 2-Brompropionylbromid in 30 ml Toluol wurde tropfenweise zu einer Lösung von 10,1 g Triethylamin und 16,5 g 2-(N-Methylbenzylamino)-ethanol in 150 ml Toluol hinzugefügt. Nach der Zugabe wurde die Mischung weiter 2 h bei Raumtemperatur gerührt. Die Mischung wurde nacheinander mit einer 1-proz. wäßrigen Natriumhydroxid-Lösung und mit Wasser gewaschen. Nach dem Trocknen wurde das Toluol unter vermindertem Druck abdestilliert, wonach 29,0 g 2-Brom-propionsäure-2-(N-methylbenzylamino)-ethylester in Form eines farblosen Öles erhalten wurden. Brechungsindex: $n_D^{20}$ = 1,5303.

Nach dem im Beispiel 1 angegebenen Verfahren wurden auch die in der folgenden Tabelle 1 aufgeführten Verbindungen der Formel (III) hergestellt.

Nit 159

## Tabelle 1

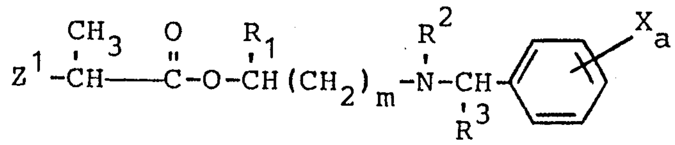

(III)

| Verbindung Nr. | $Z^1$ | $R^1$ | $R^2$ | $R^3$ | m | $X_a$ | Physikalische Konstante |
|---|---|---|---|---|---|---|---|
| III-2 | Br | H | $-C_3H_7$-iso | H | 1 | H | $n_D^{20}$ 1,5278 |
| III-3 | Br | H | $-CH_3$ | $-CH_3$ | 1 | H | |
| III-4 | Br | H | $-CH_3$ | H | 1 | 2-F | |
| III-5 | Br | H | $-CH_2CH=CH_2$ | H | 1 | 4-Cl | |
| III-6 | Br | H | $-CH_3$ | H | 1 | $4-OCH_3$ | |
| III-7 | Cl | H | $-C_3H_7$-iso | H | 1 | $3,4-Cl_2$ | |
| III-8 | Cl | H | $-CH_3$ | H | 1 | 4-Cl | |
| III-9 | Cl | $-CH_3$ | $-CH_3$ | H | 1 | H | |
| III-10 | Cl | H | $-CH_3$ | H | 2 | H | |

Beispiel 2 (Zwischenprodukt)

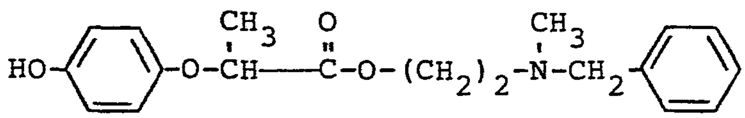

(Verbindung Nr. VII-1)

12,1 g Hydrochinon wurden in 60 ml trockenem Dimethylformamid gelöst, und unter Einblasen von Stickstoff-Gas in die Lösung wurden 31,7 g Kaliumcarbonat hinzugefügt. Unter Rühren wurde die Mischung 1 h auf 90°C erhitzt. Die Temperatur im Inneren des Reaktors wurde dann auf 60°C erniedrigt, und 30 g 2-Brompropionsäure-2-(N-methylbenzylamino)-ethylester wurden bei dieser Temperatur tropfenweise hinzugegeben. Nach der Zugabe wurde die Mischung weiter 1 h auf 90°C erhitzt. Die Reaktionsmischung wurde auf Raumtemperatur abgekühlt und dann in Eiswasser gegossen. Der pH der Mischung wurde auf 7 eingestellt, und sie wurde mit 100 ml Chloroform extrahiert. Das Chloroform wurde abdestilliert, wonach 24,0 g 2-(4-Hydroxyphenoxy)-propionsäure-2-(N-methylbenzylamino)-ethylester in Form eines blaßgelben viskosen Öles erhalten wurden:
$n_D^{25}$ = 1,5487.

Nach dem im Beispiel 2 angegebenen Verfahren wurden auch die in der folgenden Tabelle 2 aufgeführten Verbindungen der Formel (VII) hergestellt.

Tabelle 2

| Verbindung Nr. | Bezeichnung der Verbindung |
|---|---|
| VII-2 | 2-(4-Hydroxy-phenoxy)-propionsäure-2-(N-isopropyl-benzylamino)-ethylester |
| VII-3 | 2-(4-Hydroxy-phenoxy)-propionsäure-2-(N-methyl-α-methyl-benzyl-amino)-ethylester |
| VII-4 | 2-(4-Hydroxy-phenoxy)-propionsäure-2-(N-methyl-2-fluor-benzyl-amino)-ethylester |
| VII-5 | 2-(4-Hydroxy-phenoxy)-propionsäure-2-(N-allyl-4-chlorbenzyl-amino)-ethylester |
| VII-6 | 2-(4-Hydroxy-phenoxy)-propionsäure-2-(N-methyl-4-methoxy-benzyl-amino)-ethylester |
| VII-7 | 2-(4-Hydroxy-phenoxy)-propionsäure-2-(N-isopropyl-3,4-dichlorbenzyl-amino)-ethylester |
| VII-8 | 2-(4-Hydroxy-phenoxy)-propionsäure-2-(N-methyl-4-chlorbenzyl-amino)-ethylester [$n_D^{25}$ = 1,5513] |

T a b e l l e  2  (Fortsetzung)

| Verbindung Nr. | Bezeichnung der Verbindung |
|---|---|
| VII-9 | 2-(4-Hydroxy-phenoxy)-propionsäure-1-methyl-2-(N-methyl-benzyl-amino)-ethylester |
| VII-10 | 2-(4-Hydroxy-phenoxy)-propionsäure-3-(N-methyl-benzylamino)-propylester |

Beispiel 3

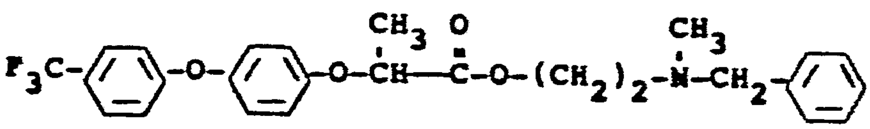

(Verbindung Nr. 1)

Eine Mischung aus 25,4 g 4-(4-Trifluormethylphenoxy)-phenol, 14,5 g wasserfreiem Kaliumcarbonat, 30,0 g 2-Brompropionsäure-2-(N-methylbenzylamino)-ethylester und 150 ml trockenem Acetonitril wurde unter kräftigem Rühren 3 h zum Rückfluß erhitzt. Nach der Reaktion wurde das Acetonitril unter vermindertem

Druck abdestilliert. Toluol (150 ml) wurde zu dem Rückstand hinzugefügt. Die Toluol-Schicht wurde nacheinander mit einer 10 %-igen wäßrigen Natriumhydroxid-Lösung und mit Wasser gewaschen. Das Toluol wurde unter vermindertem Druck abdestilliert, wodurch 41,6 g 2-/4-(4-Trifluormethyl-phenoxy)-phenoxy/-propionsäure-2-(N-methyl-benzylamino)-ethylester in Form eines farblosen Öles erhalten wurden.
$n_D^{20}$ = 1,5350.

Beispiel 4

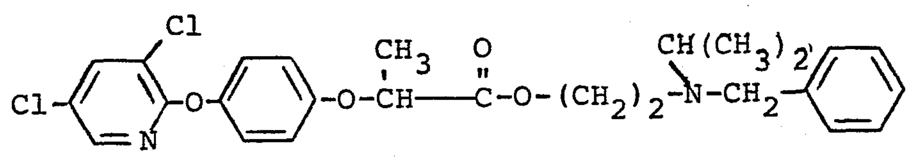

(Verbindung Nr. 2)

Eine Lösung von 34,7 g 2-/4-(3,5-Dichloro-2-pyridyloxy)phenoxy/propionylchlorid in 80 ml Toluol wurde tropfenweise bei 0°C bis 10°C zu einer Lösung von 19,3 g 2-(N-Isopropyl-benzyl-amino)-ethanol und 10,1 g Triethylamin in 100 ml Toluol hinzugefügt. Nach der Zugabe wurde die Mischung weiter 1 h bei 30°C bis 40°C gerührt. Die Mischung wurde nacheinander mit einer 1 %-igen wäßrigen Natriumhydroxid-Lösung und mit Wasser gewaschen und dann getrocknet. Nach dem Abziehen des Toluols unter vermindertem Druck

wurden 45,8 g 2-/4-(3,5-Dichlor-pyridyl-2-oxy)-phenoxy7-propionsäure-2-(N-isopropyl-benzyl-amino)-ethylester in Form eines farblosen Öles erhalten.
$n_D^{20}$ = 1,5773.

Beispiel 5

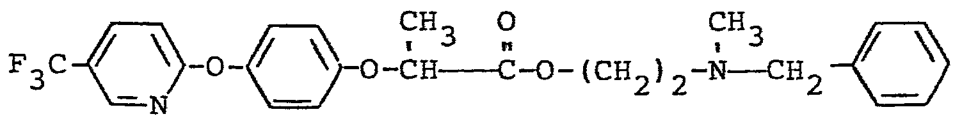

(Verbindung Nr. 3)

32,9 g 2-(4-Hydroxy-phenoxy)-propionsäure-2-(N-methyl-benzylamino)-ethylester wurden in 100 ml Dimethylsulfoxid gelöst, und 15,2 g Kaliumcarbonat wurden zugesetzt. Unter Rühren wurde die Mischung 1 h auf 90°C erhitzt. Dann wurden bei 80°C bis 90°C 20,0 g 2-Chlor-5-trifluormethylpyridin hinzugefügt. Nach der Zugabe wurde die Mischung weitere 2 h bei dieser Temperatur gerührt und dann auf Raumtemperatur abgekühlt. Die Mischung wurde in Eiswasser gegossen und mit Chloroform extrahiert. Die Chloroform-Schicht wurde entwässert, und das Chloroform wurde abdestilliert, wonach 37,5 g 2-/4-(5-Trifluormethyl-pyridyl-2-oxy)-phenoxy7-propionsäure-2-(N-methyl-benzylamino)-ethylester als blaßgelbes viskoses Öl erhalten wurden.
$n_D^{20}$ = 1,5360.

Nit 159

Nach der im Beispiel 3 angegebenen Methode wurden auch die erfindungsgemäßen Verbindungen 4 bis 8 hergestellt. Die erfindungsgemäßen Verbindungen 9 und 10 wurden nach der im Beispiel 4 beschriebenen Methode synthetisiert. Die erfindungsgemäßen Verbindungen 11 bis 14 wurden nach der Methode hergestellt, die im Beispiel 5 angegeben ist.

Die Verbindungen sind in der folgenden Tabelle 3 aufgeführt.

Nit 159

T a b e l l e 3

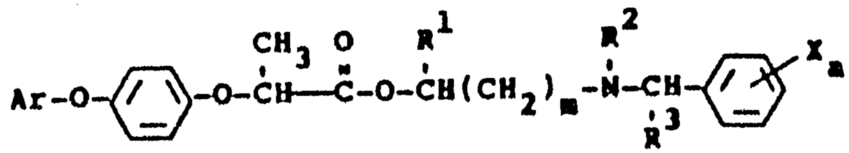 (I)

| Verbindung Nr. | Ar | $R^1$ | $R^2$ | $R^3$ | m | $X_m$ | Physikalische Konstante |
|---|---|---|---|---|---|---|---|
| 4 | 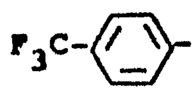 | H | $-C_3H_7$-iso | H | 1 | H | $n_D^{20}$ 1,5336 |
| 5 | 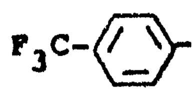 | H | $-CH_3$ | $-CH_3$ | 1 | H | $n_D^{20}$ 1,5364 |
| 6 | 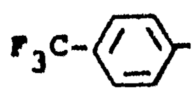 | H | $-CH_3$ | $-CH_3$ | 1 | 2-F | $n_D^{20}$ 1,5292 |
| 7 | 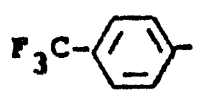 | H | $-CH_2CH=CH_2$ | H | 1 | 4-Cl | $n_D^{20}$ 1,5426 |
| 8 | 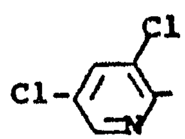 | H | $-CH_3$ | H | 1 | H | $n_D^{20}$ 1,5793 |
| 9 | 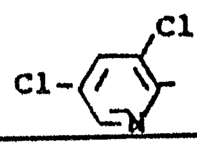 | H | $-CH_3$ | $-CH_3$ | 1 | H | $n_D^{20}$ 1,5805 |

Nit 159

Tabelle 3 (Fortsetzung)

| Verbindung Nr. | Ar | $R^1$ | $R^2$ | $R^3$ | m | $X_m$ | Physikalische Konstante |
|---|---|---|---|---|---|---|---|
| 10 | 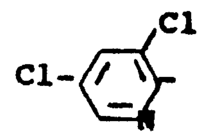 | H | $-CH_3$ | H | 1 | 2-F | $n_D^{20}$ 1,5743 |
| 11 | 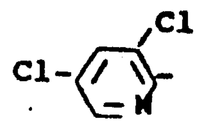 | H | $-CH_2CH=CH_2$ | H | 1 | 4-Cl | $n_D^{20}$ 1,5826 |
| 12 | 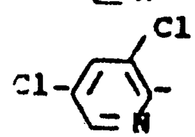 | H | $-CH_3$ | H | 1 | $4-OCH_3$ | $n_D^{20}$ 1,5835 |
| 13 | 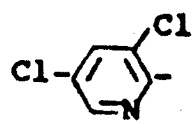 | H | $-C_3H_7$-iso | H | 1 | $3,4-Cl_2$ | $n_D^{20}$ 1,5829 |
| 14 | 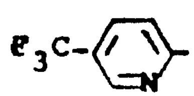 | H | $-CH_3$ | H | 1 | 4-Cl | $n_D^{20}$ 1,5395 |

Zusätzlich zu den in der Tabelle 3 aufgeführten Stoffen wurden auch die in der folgenden Tabelle 4 angegebenen erfindungsgemäßen Verbindungen nach den zuvor beschriebenen Methoden hergestellt.

Tabelle 4

| Verbindung Nr. | Bezeichnung der Verbindung |
|---|---|
| 15 | 2-[4-(4-Trifluormethyl-phenoxy)-phenoxy]-propionsäure-2-(N-methyl-benzylamino)-ethylester-hydrochlorid |
| 16 | 2-[4-(3,5-Dichlor-pyridyl-2-oxy)-phenoxy]-propionsäure-2-(N-isopropyl-benzylamino)-ethylester-hydrochlorid |
| 17 | 2-[4-(5-Trifluormethyl-pyridyl-2-oxy)-phenoxy]-propionsäure-1-methyl-2-(N-methyl-benzylamino)-ethylester |
| 18 | 2-[4-(5-Trifluormethyl-pyridyl-2-oxy)-phenoxy]-propionsäure-3-(N-methyl-benzylamino)-propylester |
| 19 | 2-[4-(3-Chlor-5-trifluormethyl-pyridyl-2-oxy)-phenoxy]-propionsäure-2-(N-methyl-benzylamino)-ethylester |

0120393

Die in der obigen Tabelle 4 aufgeführten Verbindungen 15 und 16 lassen sich in einfacher Weise durch Behandlung der erfindungsgemäßen Verbindungen 1 und 2 mit Salzsäure herstellen.

In den folgenden Beispielen werden die erfindungsgemäßen Verbindungen jeweils durch die in Klammern angegebene Zahl bezeichnet, die den Herstellungsbeispielen zu entnehmen ist. Hinweise auf "Teile" bedeuten jeweils Gewichtsteile.

Formulierungsbeispiele

Beispiel 6

Benetzbares Pulver.

15 Teile der erfindungsgemäßen Verbindung Nr. 1, 80 Teile eines Gemisches (1:5) auf Weißruß (feinpulvrigem wasserhaltigen amorphen Siliciumoxid) und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf Unkräuter und/ oder ihren Lebensraum aufgebracht.

Beispiel 7

Emulgierbares Konzentrat.

30 Teile der erfindungsgemäßen Verbindung Nr. 2, 55 Teile Xylol, 8 Teile Polyoxyethylen-alkylphenylether

und 7 Teile Calciumalkylbenzolsulfonat werden unter Rühren miteinander vermischt, wodurch ein emulgierbares Konzentrat hergestellt wird. Dieses wird mit Wasser verdünnt und auf Unkräuter und/oder ihren Lebensraum aufgebracht.

Beispiel 8

Stäubemittel.

2 Teile der erfindungsgemäßen Verbindung Nr. 2 und 98 Teile Tonpulver werden pulverisiert und gemischt, wodurch ein Stäubemittel hergestellt wird. Dieses wird auf Unkräuter und/oder ihren Lebensraum aufgebracht.

Beispiel 9

Stäubemittel.

Die erfindungsgemäße Verbindung Nr. 4 (1,5 Teile), 0,5 Teile Isopropylhydrogenphosphat und 98 Teile Tonpulver wurden pulverisiert und gemischt, wodurch ein Stäubemittel hergestellt wurde. Dieses wird auf Unkräuter und/oder ihren Lebensraum aufgebracht.

Beispiel 10

Granulat.

Wasser (25 Teile) wird zu einer Mischung aus 10 Teilen der erfindungsgemäßen Verbindung Nr. 5, 30 Teile Bentonit (Montmorillonit), 58 Teile Talkum und 2 Teile Ligninsulfonat zugesetzt, und das Gemisch wird gut geknetet.

Die Mischung wird mittels eines Extruder-Granulators zu einem Granulat mit einer Korngröße von 0,43 bis 2,0 mm (10 bis 40 mesh) verarbeitet und bei 40°C bis 50°C getrocknet, wodurch ein Granulat gebildet wird. Dieses wird auf Unkräuter und/oder ihren Lebensraum aufgebracht.

Beispiel 11

Granulat.

Ein Drehmischer wird mit 95 Teilen Tonmineral-Teilchen mit einer Teilchengrößen-Verteilung im Bereich von 0,2 bis 2 mm beschickt, und unter Drehen des Mischers werden 5 Gew.-Teile der öligen erfindungsgemäßen Verbindung Nr. 6 zur gleichmäßigen Benetzung auf die Tonmineral-Teilchen aufgesprüht. Das erhaltene Granulat wird auf Unkräuter und/oder ihren Lebensraum aufgebracht.

Die ausgezeichnete Wirkung der erfindungsgemäßen Stoffe der Formel (I) ist aus den Ergebnissen der im Folgenden beschriebenen Tests zu entnehmen.

In diesen Verwendungsbeispielen wurde die nachstehend angegebene Verbindung als Vergleichssubstanz eingesetzt:

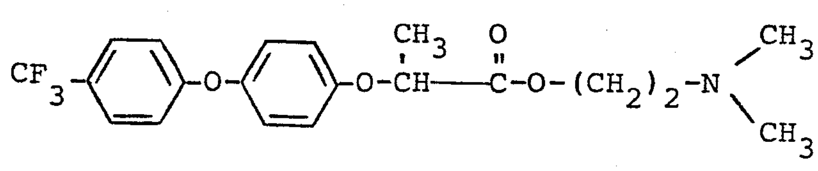

(A-1)

(Bekannt aus DE-OS 2 617 804).

0120393

Beispiel 12

Test der Vorauflauf-Bodenbehandlung an Acker-Unkräutern und-Nutzpflanzen:

Herstellung der Wirkstoffzubereitungen

| | | |
|---|---|---|
| Träger : | 5 Gew.-Teile | Aceton; |
| Emulgator: | 1 Gew.-Teil | Benzyloxypolyglykol-ether. |

Zur Herstellung eines emulgierbaren Konzentrates wurde jeweils 1 Gewichtsteil an Wirkstoff mit den oben angegebenen Mengen an Träger und Emulagor vermischt. Eine jeweils gewünschte Menge an emulgierbarem Konzentrat wurde mit Wasser verdünnt.

Test-Methode

In einem Gewächshaus wurden Samen von Erdnüssen, Garten-Erbsen, Sojabohnen und Baumwolle in Töpfe (1000 cm²) eingesät, die mit Ackerboden gefüllt waren. Boden, der Samen von Agropyron repens, Echinochloa crus-galli P. Beauv. und Setaria lutescens enthielt, wurde in einer Tiefe von 1 cm darüber gegeben.

Einen Tag nach der Einsaat und dem Bedecken mit dem Boden wurden jeweils 10 ml Wirkstoffzubereitung mit einer Wirkstoffkonzentration von 200 ppm gleichmäßig auf die Oberflächenschicht des Bodens in jedem der Test-Töpfe aufgesprüht.

Vier Wochen nach Aufsprühen der Wirkstoffzubereitung wurden die herbizide Wirkung und die Phytotoxizität der Test-Präparate untersucht.

Bewertung der herbiziden Wirkung (Unkrautvernichtungsrate bezogen auf die unbehandelten Flächen):

| | | | |
|---|---|---|---|
| 10 | | 100 % | (vollständig verdorrt) |
| 9 | mindestens | 90 %, | jedoch weniger als 100 % |
| 8 | mindestens | 80 %, | jedoch weniger als 90 % |
| 7 | mindestens | 70 %, | jedoch weniger als 80 % |
| 6 | mindestens | 60 %, | jedoch weniger als 70 % |
| 5 | mindestens | 50 %, | jedoch weniger als 60 % |
| 4 | mindestens | 40 %, | jedoch weniger als 50 % |
| 3 | mindestens | 30 %, | jedoch weniger als 40 % |
| 2 | mindestens | 20 %, | jedoch weniger als 30 % |
| 1 | mindestens | 10 %, | jedoch weniger als 20 % |
| 0 | weniger als | 10 % | (keine herbizide Wirkung) |

Bewertung der Phytotoxizität (Phytotoxizitätsrate bezogen auf die unbehandelten Flächen):

| | | | |
|---|---|---|---|
| 10 | mindestens | 90 % | (Ausrottung) |
| 9 | mindestens | 80 %, | jedoch weniger als 90 % |
| 8 | mindestens | 70 %, | jedoch weniger als 80 % |
| 7 | mindestens | 60 %, | jedoch weniger als 70 % |
| 6 | mindestens | 50 %, | jedoch weniger als 60 % |
| 5 | mindestens | 40 %, | jedoch weniger als 50 % |
| 4 | mindestens | 30 %, | jedoch weniger als 40 % |
| 3 | mindestens | 20 %, | jedoch weniger als 30 % |
| 2 | mindestens | 10 %, | jedoch weniger als 20 % |
| 1 | mehr als | 0 %, | jedoch weniger als 10 % |
| 0 | | 0 % | (keine Phytotoxizität) |

Die Testergebnisse sind in der Tabelle 5 aufgeführt.

Nit 159

Tabelle 5

| Wirkstoff Nr. | Aufwandmenge an Wirkstoff (kg/ha | Herbzide Wirkung Unkraut | | | Phytotoxitzität Kulturpflanze | | | |
|---|---|---|---|---|---|---|---|---|
| | | Agropyron repens | Echinochloa crus-galli | Setaria lutescens | Erdnüsse | Gartenbohnen | Baumwolle | Sojabohnen |
| 1 | 0,2 | 10 | 10 | 10 | 0 | 0 | 0 | 0 |
| 8 | 0,2 | 10 | 10 | 10 | 0 | 0 | 0 | 0 |
| 14 | 0,2 | 10 | 10 | 10 | 0 | 0 | 0 | 0 |
| 15 | 0,2 | 10 | 10 | 10 | 0 | 0 | 0 | 0 |
| 18 | 0,2 | 10 | 10 | 10 | 0 | 0 | 0 | 0 |
| Vergleichssubstanz | | | | | | | | |
| A-1 | 0,2 | 3 | 3 | 3 | 0 | 0 | 0 | 0 |

Beispiel 13

Test der Stengel- und Laub-Behandlung an Acker-Unkräutern und -Nutzpflanzen.

Im einem Gewächshaus wurden Samen von Sojabohnen, Rettichen und Rüben in Töpfe (2000 $cm^2$) eingesät, die mit Ackerboden gefüllt waren. Boden der Samen von Echinochloa crus-galli, Digitaria adscendens Henr., Eleusine Indica Gaertn., Setaria viridis P. Beauv., Avena fatua und Alopecurus aequalis var. amurensis Ohwi enthielt, wurde in einer Tiefe von 1 cm darüber gegeben.

Zehn Tage nach der Einsaat und dem Bedecken mit dem Boden (als die Unkräuter sich im Durchschnitt in ihrem Zweiblatt-Stadium und Rettich-, Sojabohnen- und Rübenpflanzen sich im frühen Stadium der Entwicklung ihrer Hauptblätter befanden) wurden jeweils 20 ml Wirkstoffzubereitung mit einer Wirkstoffkonzentration von 500 ppm, die in gleicher Weise wie in Beispiel 12 hergestellt worden war, gleichmäßig auf die Stengel und Blätter der Pflanzen in den Test-Töpfen gesprüht.

Drei Wochen nach dem Sprühen wurden die herbizide Wirkung und die Phytotoizität der Test-Verbindungen in der gleichen Weise wie in dem Beispiel 12 untersucht.

Die Ergebnisse sind in der Tabelle 6 aufgeführt.

0120393

Tabelle 6

| Wirkstoff Nr. | Aufwandmenge an Wirkstoff (kg/ha) | Herbizide Wirksamkeit Unkräuter | | | | | | Phytotoxizität Kulturpflanzen | | |
|---|---|---|---|---|---|---|---|---|---|---|
| | | Echinochloa crus-galli | Digitaria adscendens | Eleusine indica | Setaria virids | Avena fatua | Alopecurus aequalis | Soja-bohnen | Rettich | Rüben |
| 4 | 0,05 | 10 | 10 | 10 | 10 | 10 | 10 | 0 | 0 | 0 |
| 7 | 0,05 | 10 | 10 | 10 | 10 | 10 | 10 | 0 | 0 | 0 |
| 10 | 0,05 | 10 | 10 | 10 | 10 | 10 | 10 | 0 | 0 | 0 |
| 11 | 0,05 | 10 | 10 | 10 | 10 | 10 | 10 | 0 | 0 | 0 |
| 13 | 0,05 | 10 | 10 | 10 | 10 | 10 | 10 | 0 | 0 | 0 |
| 16 | 0,05 | 10 | 10 | 10 | 10 | 10 | 10 | 0 | 0 | 0 |
| 17 | 0,05 | 10 | 10 | 10 | 10 | 10 | 10 | 0 | 0 | 0 |
| 19 | 0,05 | 10 | 10 | 10 | 10 | 10 | 10 | 0 | 0 | 0 |
| Vergleichs-substanz A-1 | 0,05 | 2 | 2 | 1 | 2 | 0 | 2 | 0 | 0 | 0 |

Beispiel 14

Test der herbiziden Wirkung und der Hemmwirkung auf das Wiederauflaufen von Cynodon dactylon Persoon.

Eine landwirtschaftlich genutzte Ackerfläche, auf der Cynodon dactylon in Büscheln wuchs, wurde in Teilflächen von jeweils $1m^2$ unterteilt. Pro Flächeneinheit wurden jeweils 100 ml Wirkstoffzubereitung mit einer Wirkstoffkonzentration von 100 ppm auf die Stengel und Blätter von Cynodon dactylon gesprüht. 20 Tage nach dem Sprühen wurde die herbizide Wirkung nach der im Beispiel 12 angegebenen Weise ermittelt. Die Hemmwirkung auf das Wiederauflaufen von Cynodon dactylon wurde 40 beziehungsweise 80 Tage nach der Behandlung ermittelt.

Nit 159

Bewertung der Hemmwirkung auf den Wiederauflauf (Rate der Hemmwirkung auf den Wiederauflauf bezogen auf die unbehandelten Flächen):

| | | |
|---|---|---|
| 10 | | 100 % (vollständige Hemmung) |
| 9 | mindestens | 90 %, jedoch weniger als 100 % |
| 8 | mindestens | 80 %, jedoch weniger als 90 % |
| 7 | mindestens | 70 %, jedoch weniger als 80 % |
| 6 | mindestens | 60 %, jedoch weniger als 70 % |
| 5 | mindestens | 50 %, jedoch weniger als 60 % |
| 4 | mindestens | 40 %, jedoch weniger als 50 % |
| 3 | mindestens | 30 %, jedoch weniger als 40 % |
| 2 | mindestens | 20 %, jedoch weniger als 30 % |
| 1 | mindestens | 10 %, jedoch weniger als 20 % |
| 0 | weniger als | 10 % (keine Hemmwirkung) |

Die Ergebnisse sind in der Tabelle 7 aufgeführt.

Nit 159

Tabelle 7

| Verbindung Nr. | Menge des Wirkstoffs (kg/ha) | Herbizide Wirkung 10 Tage nach dem Sprühen | Hemmwirkung auf das Wiederauflaufen 40 Tage nach dem Sprühen | Hemmwirkung auf das Wiederauflaufen 60 Tage nach dem Sprühen |
|---|---|---|---|---|
| 2 | 0,1 | 10 | 9 | 9 |
| 3 | 0,1 | 10 | 10 | 9 |
| 5 | 0,1 | 10 | 10 | 9 |
| 6 | 0,1 | 10 | 10 | 9 |
| 8 | 0,1 | 10 | 9 | 9 |
| 9 | 0,1 | 10 | 10 | 9 |
| 12 | 0,1 | 10 | 9 | 8 |
| 14 | 0,1 | 10 | 10 | 9 |
| Vergleich A-1 | 0,1 | 3 | 0 | 0 |

Patentansprüche

1. Substituierte Phenoxypropionsäureester der Formel

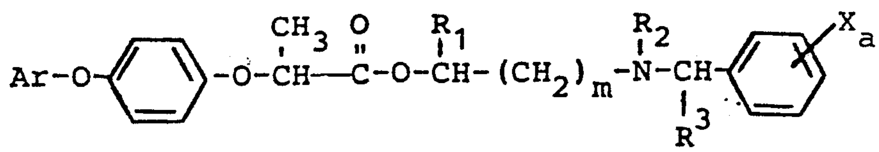

(I)

in welcher

Ar für die Reste der Formeln

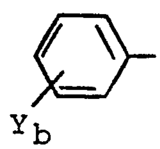

oder

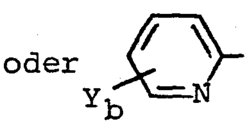

steht,

worin

Y für Halogen oder Trifluormethyl steht und

b für 1 oder 2 steht,

$R^1$ und $R^3$ unabhängig voneinander für Wasserstoff oder Methyl stehen,

$R^2$ für niederes Alkyl oder niederes Alkenyl steht,

X für Wasserstoff, Halogen oder niederes Alkoxy steht,

m für 1 oder 2 steht und

a für 1 oder 2 steht.

2. Substituierte Phenoxypropionsäureester der Formel (I), in denen

Ar für die Reste der Formeln

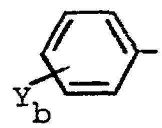 oder 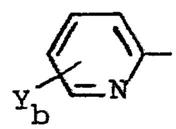 steht, worin

Y für Fluor, Chlor, Brom und/oder für Trifluormethyl steht und

b für 1 oder 2 steht,

$R^1$ und $R^3$ unabhängig voneinander für Wasserstoff oder Methyl stehen,

$R^2$ für Alkyl mit 1 bis 4 Kohlenstoffatomen oder für Alkenyl mit 2 oder 3 Kohlenstoffatomen steht,

X für Wasserstoff, Fluor, Chlor, Brom oder Alkoxy mit 1 bis 4 Kohlenstoffatomen steht

m für 1 oder 2 steht und

a für 1 oder 2 steht.

3. Verfahren zur Herstellung von substituierten Phenoxypropionsäureestern der Formel

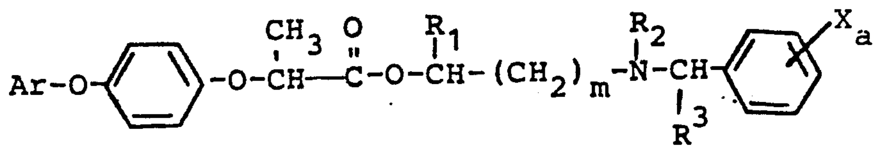

(I)

in welcher

Ar für die Reste der Formeln

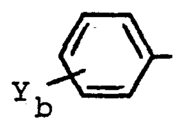 oder 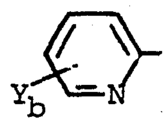 steht,

worin

Y für Halogen oder Trifluormethyl steht

und

b für 1 oder 2 steht,

$R^1$ und $R^3$ unabhängig voneinander für Wasserstoff oder Methyl stehen,

$R^2$ für niederes Alkyl oder niederes Alkenyl steht,

X für Wasserstoff, Halogen oder niederes Alkoxy steht,

m für 1 oder 2 steht und

a für 1 oder 2 steht,

dadurch gekennzeichnet, daß man

a) Verbindungen der Formel

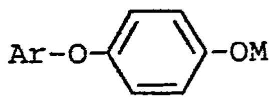 (II)

in welcher

Ar die oben angegebene Bedeutung hat

und

M für Wasserstoff oder ein Alkalimetallatom steht,

mit Verbindungen der Formel

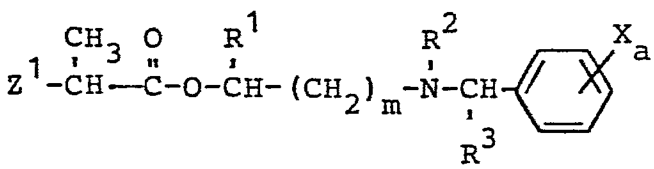 (III)

in welcher

$R^1$, $R^2$, $R^3$, X, a und m die oben angegebenen Bedeutungen haben und

$Z^1$ für Halogen steht,

umsetzt,

oder

b) Verbindungen der Formel

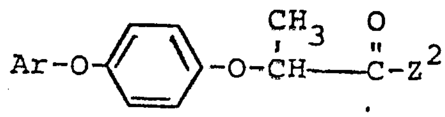

(IV)

in welcher

Ar die oben angegebene Bedeutung hat und

$Z^2$ für Hydroxy oder Halogen steht

mit Verbindungen der Formel

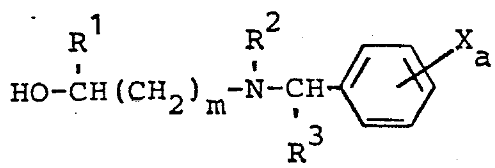

(V)

in welcher

$R^1$, $R^2$, $R^3$, X, a und m die oben angegebenen Bedeutungen haben,

umsetzt,

oder

c) Verbindungen der Formel

$$Ar-Z^1 \qquad (VI)$$

in welcher

Ar und $Z^1$ die oben angegebenen Bedeutungen haben,

mit Verbindungen der Formel

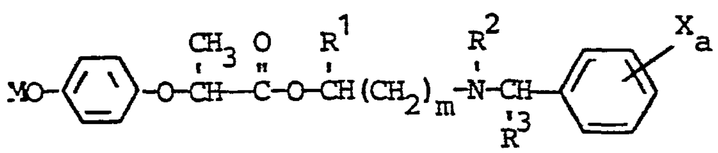

(VII)

in welcher

$R^1$, $R^2$, $R^3$, X, M, a und m die oben angegebenen Bedeutungen haben,

umsetzt.

4. Herbizide Mittel gekennzeichnet durch einen Gehalt an mindestens einem substituierten Phenoxypropionsäureester der Formel (I).

5. Verwendung von substituierten Phenoxypropionsäureestern der Formel (I) zur Bekämpfung von Unkräutern.

6. Verfahren zur Bekämpfung von Unkräutern, dadurch gekennzeichnet, daß man substituierte Phenoxypropionsäureester der Formel (I) auf die Unkräuter und/oder ihren Lebensraum ausbringt.

7. Verbindungen der Formel

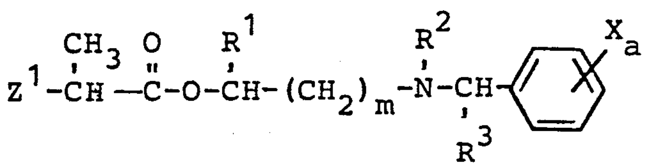

(III)

in welcher

$R^1$ und $R^3$ unabhängig voneinander für Wasserstoff oder Methyl stehen,

$R^2$ für niederes Alkyl oder niederes Alkenyl steht,

X für Wasserstoff, Halogen oder niederes Alkoxy steht,

m für 1 oder 2 steht,

a für 1 oder 2 steht und

$Z^1$ für Halogen steht.

8. Verfahren zur Herstellung von Verbindungen der Formel

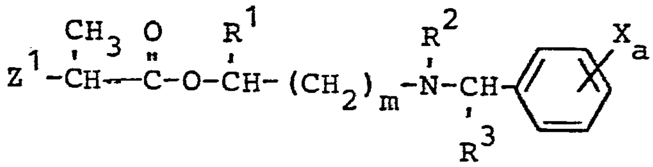

(III)

in welcher

$R^1$ und $R^3$ unabhängig voneinander für Wasserstoff oder Methyl stehen,

$R^2$ für niederes Alkyl oder niederes Alkenyl steht,

X für Wasserstoff, Halogen oder niederes Alkoxy steht,

m für 1 oder 2 steht,

a für 1 oder 2 steht und

$Z^1$ für Halogen steht,

dadurch gekennzeichnet, daß man Verbindungen der Formel

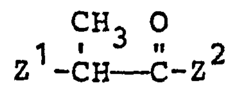

(VIII)

in welcher

$Z^1$ die oben angegebene Bedeutung hat

und

$Z^2$ für Hydroxy oder Halogen steht,

mit Verbindungen der Formel

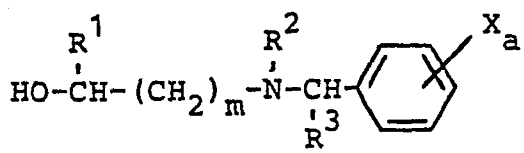

(V)

in welcher

$R^1$, $R^2$, $R^3$, X, a und m die oben angegebenen Bedeutungen haben,

umsetzt.

9. Verbindungen der Formel

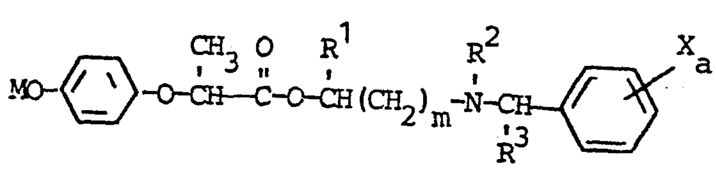

(VII)

in welcher

$R^1$ und $R^3$ unabhängig voneinander für Wasserstoff oder Methyl stehen,

$R^2$ für niederes Alkyl oder niederes Alkenyl steht,

X für Wasserstoff, Halogen oder niederes Alkoxy steht,

m für 1 oder 2 steht,

a für 1 oder 2 steht und

M für Wasserstoff oder ein Alkalimetallatom steht.

10. Verfahren zur Herstellung von Verbindungen der Formel

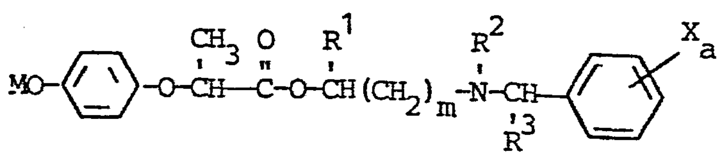

(VII)

in welcher

$R^1$ und $R^3$ unabhängig voneinander für Wasserstoff oder Methyl stehen,

$R^2$ für niederes Alkyl oder niederes Alkenyl steht,

X für Wasserstoff, Halogen oder niederes Alkoxy steht,

m für 1 oder 2 steht,

a für 1 oder 2 steht und

M für Wasserstoff oder ein Alkalimetallatom steht,

dadurch gekennzeichnet, daß man Verbindungen der Formel

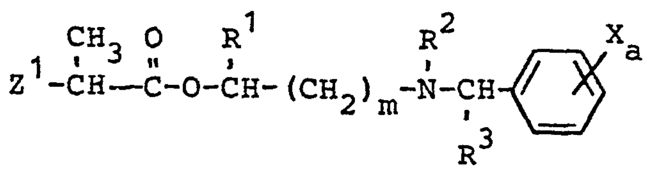

(III)

in welcher

$R^1$, $R^2$, $R^3$, X, a und m die oben angegebenen Bedeutungen haben und

$Z^1$ für Halogen steht,

mit Hydrochinon der Formel

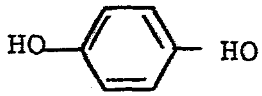

(IX)

umsetzt und gegebenenfalls die dabei entstehenden Produkte mit einem Alkalimetallhydroxid, einem Alkalimetallcarbonat oder einem Alkalimetallalkoholat umsetzt.

Europäisches Patentamt

# EUROPÄISCHER RECHERCHENBERICHT

0120393

Nummer der Anmeldung

EP 84 10 2699

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | EP-A-0 003 313 (CIBA-GEIGY) * Ansprüche; Beispiel 18 * | 1,3-7 | C 07 C 93/227<br>A 01 N 39/02<br>A 01 N 43/40 |
| | --- | | |
| X | FR-A-2 255 891 (SIEGFRIED) * Anspruch 2; Seite 5, Nr. 5674,1774 * | 7 | |
| | --- | | |
| Y | EP-A-0 068 260 (NIHON TOKUSHU NOYAKU SEIZO) * Ansprüche * | 1,3-6 | |
| | --- | | |
| Y | FR-A-2 348 908 (HOECHST) * Ansprüche * | 1,3-6 | |
| | ----- | | |

| RECHERCHIERTE SACHGEBIETE (Int. Cl. 3) |
|---|
| C 07 C 93/00<br>A 01 N 39/00<br>A 01 N 43/00 |

Der vorliegende Recherchenbericht wurde für alle Patentansprüche erstellt.

| Recherchenort | Abschlußdatum der Recherche | Prüfer |
|---|---|---|
| DEN HAAG | 26-06-1984 | MOREAU J.M. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument